# EUROPEAN PATENT APPLICATION

(11) **EP 4 699 633 A1**
(43) Date of publication of application: **25.02.2026**
(21) Application number: 24306377.3
(22) Date of filing: 20.08.2024
(51) Int. Cl.: A61M 15/00, A61M 11/00, A61M 39/20, B05B 1/00

(54) **CAP ASSEMBLY FOR A MEDICAL DEVICE**

(71) Applicant: Becton Dickinson France, 38800 Le Pont-de-Claix (FR)
(72) Inventor: DINC, Mirhac, 74960 Annecy (FR); SANGLARD, Laura, 38660 Lumbin (FR); BARRE, Julie, 38100 Grenoble (FR); PAULET, Mathilde, 38100 Grenoble (FR)
(74) Representative: Germain Maureau

(57) **Abstract**

A cap assembly (10) including a cap (20) configured to seal at least a portion of a medical device (100), the cap defining a cavity (28) and including a first coupling member (40), and a connection element (60) configured to be arranged on the medical device and to be received within the cavity, the connection element including a second coupling member (80) configured to engage the first coupling member to secure the cap to the connection element, wherein movement of the cap in a first direction relative to the connection element causes one or more of the cap and the connection element to deform to secure the cap to the connection element and movement of the cap in a second direction relative to the connection element causes one or more of the cap and the connection element to deform to remove the cap from the connection element.

## Description

### FIELD

The present disclosure relates to the field of medical devices. Particularly, the present disclosure relates to a cap assembly configured for use on a medical device.

### BACKGROUND

A medical device used to deliver a material contained in the medical device to a user by spraying, such as an ear, nose, and throat spray device and/or the like, typically includes a cap to seal and protect a nozzle of the medical device used to dispense the spray during transport and/or storage.

Currently available caps for medical devices have a tendency to become partially or fully removed during transport and/or storage of the medical device, resulting in leakage and/or contamination of the material contained in the medical device. Additionally, currently available caps for medical devices are often difficult for a user to secure to the medical device and/or to remove from the medical device.

It is desirable to provide a cap assembly configured for use on a medical device that is capable of maintaining a robust connection between a cap and the medical device during transport, storage, and usage of the medical device, while also allowing a user to easily secure and remove the cap to and from the medical device, to ensure a positive user experience of the medical device.

### SUMMARY

According to aspects of the present disclosure, a cap assembly configured for use on a medical device is provided. The cap assembly extends between a proximal end and a distal end along a longitudinal axis. The cap assembly includes a cap configured to seal at least a portion of a medical device. The cap includes a body extending axially between a proximal end and a distal end. The cap defines a cavity extending from the proximal end toward the distal end. The cap includes a first coupling member. The cap assembly includes a connection element configured to be arranged on the medical device and to be received within the cavity. The connection element includes a body extending axially between a proximal end and a distal end. The connection element includes a second coupling member configured to engage the first coupling member of the cap to secure the cap to the connection element. At least a portion of one or more of the cap and the connection element is elastically deformable. Movement of the cap in a first direction relative to the connection element causes one or more of the cap and the connection element to elastically deform to secure the cap to the connection element. Movement of the cap in a second direction relative to the connection element causes one or more of the cap and the connection element to elastically deform to remove the cap from the connection element.

According to aspects of the disclosure, the first coupling member of the cap may extend radially from the body of the cap.

According to aspects of the disclosure, the second coupling member of the connection element may extend radially from the body of the connection element.

According to aspects of the disclosure, the first coupling member of the cap may include a recess defined by the body of the cap and the second coupling member of the connection element may include a shoulder extending from the body of the connection element.

According to aspects of the disclosure, the first coupling member of the cap may include a shoulder extending from the body of the cap and the second coupling member of the connection element may include a recess defined by the body of the connection element.

According to aspects of the disclosure, the cap may be formed from a first material having a first rigidity, the connection element may be formed from a second material having a second rigidity, and the second rigidity of the second material may be greater than the first rigidity of the first material

According to aspects of the disclosure, the body of the connection element may define a chamber extending between the proximal end and the distal end of the body of the connection element.

According to aspects of the disclosure, the body of the connection element may define a first opening at the proximal end of the body of the connection element and a second opening at the distal end of the body of the connection element.

According to aspects of the disclosure, the first opening of the body of the connection element may be configured to receive at least a portion of the medical device.

According to aspects of the disclosure, the second opening of the body of the connection element may be configured to dispense a material from the medical device.

According to aspects of the disclosure, the cap may include an axially extending protuberance configured to hermetically seal the second opening of the body of the connection element.

According to aspects of the disclosure, the body of the connection element may include a first section having a first diameter, the body of the connection element may include a second section having a second diameter, and the second diameter of the second section may be greater than the first diameter of the first section.

According to aspects of the disclosure, the proximal end of the body of the cap may be configured to extend to a position about the first section of the connection element when the connection element is fully-received within the cavity of the cap.

According to aspects of the disclosure, the cap may include an aperture defined by the proximal end of the body of the cap in communication with the cavity of the cap and the first section of the connection element may be received through the aperture when the connection element is inserted into the cap.

According to aspects of the disclosure, the first coupling member of the cap may be arranged at a position on the body of the cap at a distance within a range of 5 mm to 10 mm from the proximal end of the cap.

According to aspects of the disclosure, a medical device including the cap assembly according to any aspect of the disclosure presented herein is provided.

According to aspects of the disclosure, a method of securing a cap to a medical device is provided. The method includes providing the cap assembly according to any aspect of the disclosure herein. The method includes arranging the connection element on a medical device. The method includes inserting the connection element into the cavity of the cap.

In the manner described and according to aspects illustrated herein, the cap assembly, the medical device, and the method of securing a cap to a medical device are capable of maintaining a robust connection between a cap and the medical device during transport, storage, and usage of the medical device, while also allowing a user to easily secure and remove the cap to and from the medical device, to ensure a positive user experience of the medical device.

### BRIEF DESCRIPTION OF THE DRAWINGS

Aspects of an example will be described with reference to the drawings, where like numerals represent like parts:
Figure 1 shows a front cross-sectional view of a cap assembly configured for use on a medical device according to aspects of the disclosure, depicting the cap assembly mounted on the medical device;
Figure 2A shows a schematic cross-sectional view of the cap assembly according to Figure 1;
Figure 2B shows a schematic cross-sectional view of an alternative arrangement of the cap assembly according to Figure 1;
Figure 2C shows a schematic cross-sectional view of an alternative arrangement of the cap assembly according to Figure 1;
Figure 2D shows a schematic cross-sectional view of an alternative arrangement of the cap assembly according to Figure 1; and
Figure 3 shows a front cross-sectional view of an injection molding process of the cap assembly according to Figure 1.

### DETAILED DESCRIPTION

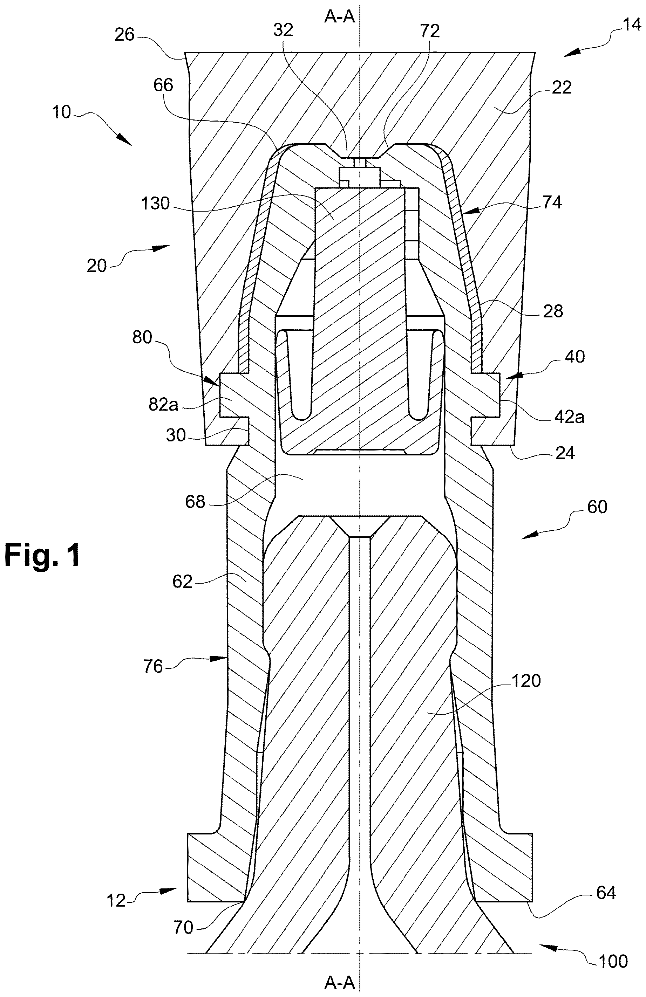

A cap assembly configured for use on a medical device according to aspects of the disclosure is described with reference to Figures 1-3. Like numerals represent like parts, and the cap assembly will generally be referred to by the reference numeral 10. Although the cap assembly 10 is described with reference to specific examples, it should be understood that modifications and changes may be made to these examples without going beyond the general scope as defined by the claims. In particular, individual characteristics of the various examples shown and/or mentioned herein may be combined in additional examples. Consequently, the description and the drawings should be considered in a sense that is illustrative rather than restrictive. The Figures, which are not necessarily to scale, depict illustrative aspects and are not intended to limit the scope of the disclosure. The illustrative aspects depicted are intended only as exemplary.

The term "exemplary" is used in the sense of "example," rather than "ideal." While aspects of the disclosure are amenable to various modifications and alternative forms, specifics thereof have been shown by way of example in the drawings and will be described in detail. It should be understood, however, that the intention is not to limit aspects of the disclosure to a particular example described. On the contrary, the intention of this disclosure is to cover all modifications, equivalents, and alternatives falling within the scope of the disclosure.

Various materials, methods of construction, methods of fastening, and the like may be described in the context of disclosed examples. Those skilled in the art will recognize known substitutes for the materials, construction methods, fastening methods, and the like, all of which are contemplated as compatible with the disclosed example and are intended to be encompassed by the appended claims.

As used in this disclosure and the appended claims, the singular forms "a," "an," and "the" include plural referents, unless the content clearly dictates otherwise. As used in this disclosure and the appended claims, the term "or" is generally employed in a sense including "and/or," unless the content clearly dictates otherwise.

Throughout the description, including the claims, the terms "comprising a," "including a," and "having a" should be understood as being synonymous with "comprising one or more," "including one or more," and "having one or more" unless otherwise stated. In addition, any range set forth in the description, including the claims, should be understood as including its end value(s), unless otherwise stated. Specific values for described elements should be understood to be within accepted manufacturing or industry tolerances known to one of skill in the art, and any use of the terms "substantially," "approximately," and "generally" should be understood to mean falling within such accepted tolerances.

When an element or feature is referred to herein as being "on," "engaged to," "connected to," or "coupled to" another element or feature, it may be directly on, engaged, connected, or coupled to the other element or feature, or intervening elements or features may be present. In contrast, when an element or feature is referred to as being "directly on," "directly engaged to," "directly connected to," or "directly coupled to" another element or feature, there may be no intervening elements or features present. Other words used to describe the relationship between elements or features should be interpreted in a like manner (e.g., "between" versus "directly between," "adjacent" versus "directly adjacent," etc.).

Spatially relative terms, such as "top," "bottom," "middle," "inner," "outer," "beneath," "below," "lower," "above," "upper," and the like may be used herein for ease of description to describe one element or relationship of a feature to another element(s) or feature(s) as illustrated in the drawings. Spatially relative terms may be intended to encompass different orientations of a device in use or operation in addition to the orientation depicted in the drawings. For example, if the device in the drawings is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

Although the terms "first," "second," "third," etc. may be used herein to describe various elements, components, regions, layers, sections, and/or parameters, these elements, components, regions, layers, sections, and/or parameters should not be limited by these terms. These terms are only used to distinguish one element, component, region, layer, section, or parameter from another element, component, region, layer, section, or parameter. Thus, a first element, component, region, layer, section, or parameter discussed herein could be termed a second element, component, region, layer, section, or parameter without departing from the teachings of the present disclosure.

As shown by Figure 1, the cap assembly 10 is configured for use on a medical device 100. It is contemplated that the medical device 100 in which the cap assembly 10 is configured for use on may be configured to deliver a material (not shown) contained in the medical device 100 to a user by spraying and/or dispensing a flow of droplets of a fluid onto a body surface of a user. In examples, the material contained within the medical device 100 may be a fluid having medicinal properties. It is contemplated that the medical device 100 may include a reservoir (not shown) configured to contain the material, a pump mechanism (not shown) configured to transport the material from the reservoir through the medical device 100, and a tip 120 configured to dispense and/or deliver the material to a user of the medical device 100.

Referring to Figure 1, the cap assembly 10 extends between a proximal end 12 and a distal end 14 along a longitudinal axis A-A. It is contemplated that the terms "proximal" and "distal" as used herein may be understood as a position and/or location corresponding to a component, device, and/or a portion thereof in relation to a hand (not shown) of the user when the user is holding the medical device 100. In examples, the proximal end 12 of the cap assembly 10 may correspond to a position and/or location provided at a distance closer to the hand of the user holding the medical device 100 than the distal end 14 of the cap assembly 10. The distal end 14 of the cap assembly 10 may correspond to a position and/or location provided at a distance that is farther from the hand of the user holding the medical device 100 than the proximal end 12 of the cap assembly 10. Additionally or alternatively, a distal direction may be understood as a direction of delivery of the material from the medical device 100 during performance of a delivery operation of the material from the medical device 100 and a proximal direction may be understood as a direction opposite to the direction of delivery of the material from the medical device 100 and/or a direction toward the hand of the user holding the medical device 100.

The cap assembly 10 includes a cap 20 configured to seal at least a portion of the medical device 100 and a connection element 60 configured to secure the cap 20 to the medical device 100. In examples, the cap 20 is configured to enclose and/or seal at least a portion of the connection element 60 to seal at least a portion of the connection element 60 and/or to be secured to at least a portion of the connection element. Additionally or alternatively, the cap 20 is configured to enclose and/or seal at least a portion of the connection element 60 to seal at least a portion of the medical device 100 and/or to be secured to the medical device 100.

The cap 20 includes a body 22 extending axially between a proximal end 24 and a distal end 26. The body 22 of the cap 20 defines a cavity 28 extending between at least the proximal end 24 and the distal end 26 of the cap 20. Additionally or alternatively, the cavity 28 extends from the proximal end 24 of the cap 20 toward the distal end 26 of the cap 20. The cavity 28 is configured to receive at least a portion of the connection element 60. The proximal end 24 of the cap 20 is configured to be open and the distal end 26 of the cap 20 is configured to be closed. In examples, the body 22 of the cap 20 defines an aperture 30 at the proximal end 24 of the cap 20. The aperture 30 is in communication with the cavity 28. The aperture 30 is configured to receive at least a portion of the connection element 60 so that the connection element 60 is capable of being received in the cavity 28. In examples, the cavity 28 has a diameter within a range of 2 mm to 7 mm. Additionally or alternatively, the aperture 30 has a diameter within a range of 4 mm to 7 mm. In this manner, the cap 20 is capable of forming a tight seal and/or surface-to-surface fit about at least a portion of the connection element 60. In examples, the cap 20 is capable of forming a hermetic seal about at least a portion of the connection element 60.

Referring to Figure 1, at least a portion of the connection element 60 is configured to be received within the cavity 28 of the cap 20. Additionally, the connection element 60 is configured to be arranged on at least a portion of the medical device 100. In particular, the connection element 60 may be configured to be arranged on at least a portion of the tip 120 of the medical device 100. In examples, the connection element 60 may be configured to be connected to the tip 120, through an interference fit, a threaded connection, an adhesive bond, and/or the like. Alternatively, the connection element 60 may be integrally formed, in one piece, with the tip 120. Accordingly, it is contemplated that the connection element 60 may be considered part of the tip 120 and, thus, the medical device 100. Additionally or alternatively, the cap 20 may be considered part of the medical device 100. However, the cap assembly 10 will be discussed herein independently of the medical device 100, unless discussion of the cap assembly 10 as part of the medical device 100 is otherwise necessary.

The connection element 60 includes a body 62 extending axially between a proximal end 64 and a distal end 66. The body 62 of the connection element 60 defines a chamber 68 extending between the proximal end 64 and the distal end 66 of the connection element 60. The chamber 68 is configured to receive at least a portion of the medical device 100. In particular, the chamber 68 may be configured to receive at least a portion of the tip 120 of the medical device 100. Additionally or alternatively, the chamber 68 may be configured to receive an internal part 130 of the medical device 100 configured to form a conduit (not shown) for the material within the chamber 68.

The proximal end 64 and the distal end 66 of the connection element 60 are configured to be open. In examples, the body 62 of the connection element 60 defines a first opening 70 at the proximal end 64 of the connection element 60 and a second opening 72 at the distal end 66 of the connection element 60. The first opening 70 and the second opening 72 are in communication with the chamber 68 of the connection element 60. The first opening 70 is configured to receive at least a portion of the medical device 100. In particular, the first opening 70 may be configured to receive at least a portion of the tip 120 of the medical device 100. In examples, the chamber 68 has a diameter within a range of 3 mm to 8 mm. Additionally or alternatively, the first opening 70 has a diameter within a range of 5 mm to 10 mm. In this manner, the connection element 60 is configured to form a secure connection with the medical device 100. The second opening 72 is configured to dispense the material from the medical device 100. In this manner, the second opening 72 is configured to control a size and/or velocity of dispersal of the material from the medical device 100. As shown by Figure 1, the cap 20 may include an axially extending protuberance 32 configured to seal the second opening 72 when the connection element 60 is received within the cavity 28 of the cap 20, thereby further enhancing the seal between the cap 20 and the connection element 60 and, thus, between the cap 20 and the medical device 100.

In examples, the body 62 of the connection element 60 includes a first section 74 and a second section 76. In examples, at least a portion of the first section 74 is configured to be received within the cavity 28 of the cap 20 and the second section 76 is configured to receive at least a portion of the tip 120 of the medical device 100. The first section 74 has a first diameter and the second section 76 has a second diameter. The second diameter of the second section 76 may be greater than the first diameter of the first section 74. In examples, the first section 74 may have a diameter within a range of 2 mm to 7 mm. The second section 76 may have a diameter within a range of 4 mm to 8 mm. In this manner, the connection element 60 is configured to secure the cap 20 to the medical device 100 without excessive deformation of the cap 20, while also being configured to receive and connect to the medical device 100. It is contemplated that the first diameter of the first section 74 and the second diameter of the second section may be understood to correspond to a diameter measured with respect to an exterior surface of the body 62 of the connection element 60.

Referring to Figures 1-2, the cap 20 and the connection element 60 are configured to be coupled to each other to secure the cap 20 to the medical device 100. The cap 20 includes a first coupling member 40 configured to secure the cap 20 to the connection element 60 and, thus, to the medical device 100. The connection element 60 includes a second coupling member 80 configured to secure the cap 20 to the connection element 60 and, thus, to the medical device 100. In this manner, the first coupling member 40 of the cap 20 and the second coupling member 80 of the connection element 60 are configured to engage each other to secure the cap 20 to the medical device 100 when the connection element 60 is received within the cavity 28 of the cap 20.

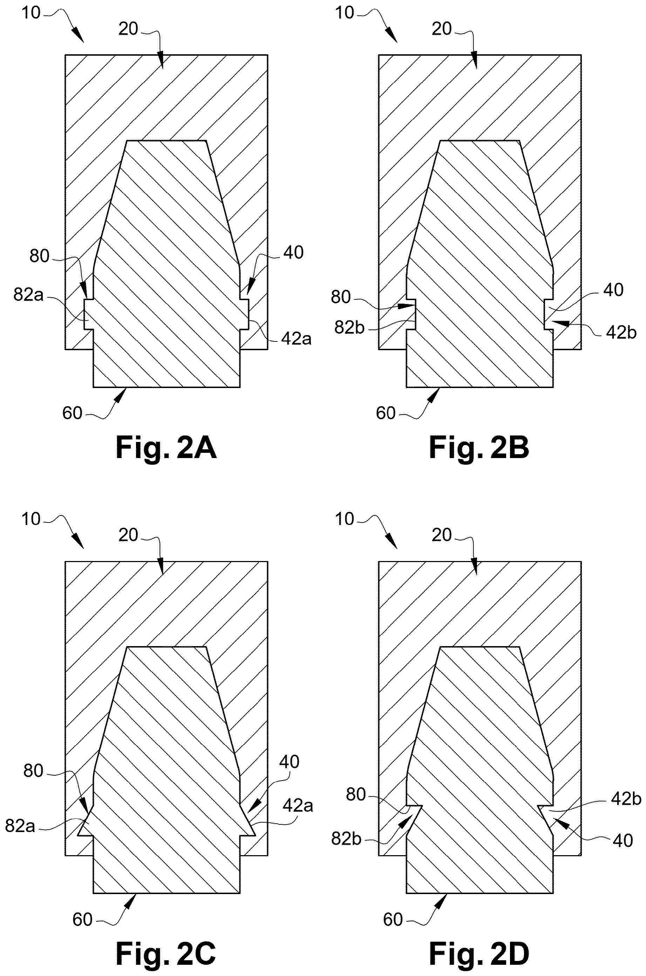

In examples, the first coupling member 40 of the cap 20 may extend radially from the body 22 of the cap 20 and the second coupling member 80 of the connection element 60 may extend radially from the body 62 of the connection element 60. Additionally or alternatively, the first coupling member 40 may be understood as extending circumferentially and/or annularly from the body 22 of the cap 20 and the second coupling member 80 may be understood as extending circumferentially and/or annularly from the body 62 of the connection element 60. As illustrated by Figures 1, 2A, and 2C, the first coupling member 40 may include a recess 42a defined by the body 22 of the cap 20 and the second coupling member 80 may include a shoulder 82a extending from the body 62 of the connection element 60. The recess 42a of the first coupling member 40 may be configured to receive the shoulder 82a of the second coupling member 80 when the connection element 60 is received within the cavity 28 of the cap 20, thereby securing the cap 20 to the connection element 60 and, thus, to the medical device 100.

Alternatively, as illustrated by Figures 2B-2C, the first coupling member 40 of the cap 20 may include a shoulder 42b extending from the body 22 of the cap 20 and the second coupling member 80 of the connection element 60 may include a recess 82b defined by the body 62 of the connection element 60. The recess 82b of the second coupling member 80 may be configured to receive the shoulder 42b of the first coupling member 40 when the connection element 60 is received within the cavity 28 of the cap 20, thereby securing the cap 20 to the connection element 60 and, thus, to the medical device 100. However, the first coupling member 40 will be described herein as including the recess 42a and the second coupling member 80 will be described herein as including the shoulder 82a, unless description of first coupling member 40 including the shoulder 42b and the second coupling member 80 including the recess 82b is otherwise necessary. Further, it is contemplated that any description of structures and/or relationships corresponding to examples in which the first coupling member 40 includes the recess 42a and the second coupling member 80 includes the shoulder 82a also correspond to examples in which the first coupling member 40 includes the shoulder 42b and the second coupling member 80 includes the recess 82b.

Referring to Figures 1 and 2A-2B, the recess 42a of the first coupling member 40 of the cap 20 may extend radially from the body 22 of the cap 20, such that a top surface of the body 22 of the cap 20 within the recess 42a extends substantially perpendicular to the axis A-A of the cap assembly 10. In such an example, the shoulder 82a of the second coupling member 80 of the connection element 60 is configured to be complimentary to the recess 42a of the first coupling member 40 and, thus, may extend radially from the body 62 of the connection element 60, such that a top surface of the shoulder 82a extends substantially perpendicular to the axis A-A. Alternatively, referring to Figures 2C-2D, the recess 42a of the first coupling member 40 may extend radially from the body 22 of the cap 20, such that the top surface of the body 22 of the cap 20 within the recess 42a is sloped with respect to the axis A-A. In such an example, the shoulder 82a of the second coupling member 80 is configured to be complimentary to the recess 42a of the first coupling member 40 and, thus, may extend radially from the body 62 of the connection element 60, such that the top surface of the shoulder 82a is sloped with respect to the axis A-A. In examples, a positive or negative angle of a slope of the top surface of the body 22 of the cap 20 within the recess 42a of the first coupling member 40 and/or the top surface of the shoulder 82a of the second coupling member 80 may be within a range of 10° to 50°, or stepped at 90°.

In examples, the cap 20 is formed from a first material having a first rigidity and the connection element 60 is formed from a second material having a second rigidity. The second rigidity of the second material of the connection element 60 may be greater than the first rigidity of the first material of the cap 20. In examples, at least a portion of one or more of the first material of the cap 20 and the second material of the connection element 60 may be an elastically deformable material. In this manner, one or more of the first coupling member 40 of the cap 20 and the second coupling member 80 of the connection element 60 is capable of flexure and/or movement.

In examples, the first material of the cap 20 is an elastically deformable material and capable of flexure and/or movement. The elastically deformable material of the first material may be one or more of rubber, silicone, and/or the like. In examples, the second material of the connection element 60 is a material having a greater rigidity than the first material of the cap 20, such as plastic, glass, and/or the like. Accordingly, the cap 20 and, thus, the first coupling member 40, is capable of flexure and/or movement relative to the connection element 60. Flexure and/or movement of the cap 20 allows the second coupling member 80 to move the first coupling member 40, such that the second coupling member 80 may be received within the first coupling member 40 when the cap 20 is pushed onto the connection element 60 and/or when the connection element 60 is pushed through and received within the cavity 28 of the cap 20. Further, flexure and/or movement of the cap 20 allows the second coupling member 80 to move the first coupling member 40, such that the second coupling member 80 may be removed from the first coupling member 40 when the cap 20 is pulled from the connection element 50 and/or when the connection element 60 is pulled out of and removed from the cavity 28 of the cap 20.

Referring to Figure 1, the body 22 of the cap 20 is configured to extend to a position about and/or within the first section 74 of the connection element 60 when the connection element 60 is fully-received within the cavity 28 of the cap 20. The proximal end 24 and, thus, the aperture 30, of the body 22 of the cap 20 is positioned about and/or within the first section 74 of the connection element 60 when the connection element 60 is fully-received within the cavity 28 of the cap 20. The first coupling member 40 may be arranged at a position at or adjacent to the proximal end 24 of the body 22 of the cap 20. In examples, the cavity 28 of the cap 20 may extend to a length within a range of 2 mm to 7 mm. The first section 74 of the connection element 60 may extend to a length within a range of 2 mm to 7 mm. The first coupling member 40 of the cap 20 may be arranged at a position on the body 22 of the cap 20 that is at distance within a range of 5 mm to 10 mm from the distal end 26 of the body 22 of the cap 20. In this manner, the body 22 of the cap 20 is prevented from extending to the second section 76 of the connection element 60, which has a greater diameter than the first section 74 of the connection element 60. Accordingly, the cap 20 is prevented from becoming excessively deformed by the second section 76 of the connection element 60 and, thus, prone to loosening and unintentional removal from the connection element 60 during transport and/or storage of the medical device 100.

To apply the connection element 60 to the medical device 100, the tip 120 of the medical device 100 is inserted, in a first direction, toward and through the first opening 70 of the connection element 60. The tip 120 is inserted through the chamber 68 of the connection element 60 to form a secure connection between the connection element 60 and the tip 120 and, thus, between the connection element 60 and the medical device 100. To apply the cap 20 to the connection element 60, the cap 20 is pushed onto the first section 74 of the connection element 60 in the first direction and the first section 74 of the connection element 60 is inserted through the aperture 30 of the cap 20. The first section 74 of the connection element 60 is inserted through the cavity 28 of the cap 20. The shoulder 82a of the second coupling member 80 of the connection element 60 applies a force on the body 22 of the cap 20, causing the body 22 of the cap 20 and, thus, the first coupling member 40 of the cap 20, to flex outwardly, allowing the second coupling member 80 to be received within the first coupling member 40. It is contemplated that the term "outward" as used herein may be understood in relation to the axis A-A of the cap assembly 10. The first section 74 of the connection element 60 is inserted through the cavity 28 until the first section 74 of the connection element 60 is fully-received within the cavity 28 of the cap 20 and/or until the second coupling member 80 is received within the first coupling member 40.

To remove the cap 20 from the connection element 60 and, thus, from the medical device 100, the medical device 100 and, thus, the connection element 60, is pulled in a second direction, away from the cap 20. Additionally or alternatively, the cap 20 is pulled in a second direction, away from the connection element 60 and, thus, the medical device 100. The shoulder 82a of the second coupling member 80 of the connection element 60 applies a force on the body 22 of the cap 20, causing the body 22 of the cap 20 and, thus, the first coupling member 40 of the cap 20, to flex outwardly, allowing the second coupling member 80 to be removed from the first coupling member 40, thereby allowing the cap 20 to be removed from the connection element 60 and, thus, the medical device 100.

In this manner, the cap assembly 10 is capable of forming a robust connection between the cap 20 and the medical device 100 during transport, storage, and usage of the medical device 100, while also allowing a user to be able to easily secure and remove the cap 20 to and from the medical device 100, to ensure a positive user experience of the medical device 100.

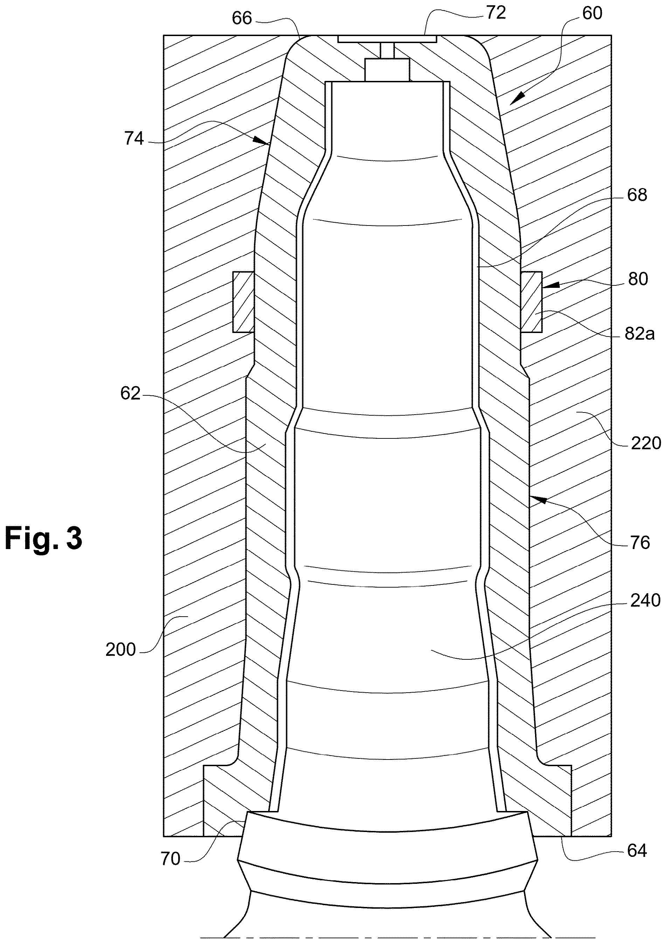

As shown by Figure 3, it is contemplated that the connection element 60 may be formed through an injection molding process. To form the connection element 60, the injection molding process may include a first molding part 200, a second molding part 220, and a core molding part 240. In examples, the first molding part 200 and the second molding part 220 may be movable and the core molding part 240 may be fixed. The core molding part 240 may be provided in a central portion of the mold and the first molding part 200 and the second molding part 220 may oppose each other at opposing sides of the core molding part 240. Once the connection element 60 has been cooled and hardened, the first molding part 200 is moved in a first direction, away from the core molding part 240, and the second molding part 220 is moved in a second direction, opposite the first direction of the first molding part 200, away from the core molding part 240, to eject the connection element 60 from the mold. In this manner, the connection element 60 is formed without producing defects, such as distortion and/or breakage of the second coupling member 80 from the body 62 of the connection element 60.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

Additionally, all of the disclosed features of an apparatus may be transposed, alone or in combination, to a method and vice versa.

## Claims

1. A cap assembly (10) configured for use on a medical device (100), the cap assembly extending between a proximal end (12) and a distal end (14) along a longitudinal axis (A-A), the cap assembly comprising:
a cap (20) configured to seal at least a portion of a medical device (100), the cap including a body (22) extending axially between a proximal end (24) and a distal end (26), the cap defining a cavity (28) extending from the proximal end toward the distal end, and the cap including a first coupling member (40); and
a connection element (60) configured to be arranged on the medical device (100) and to be received within the cavity (28) of the cap (20), the connection element including a body (62) extending axially between a proximal end (64) and a distal end (66), and the connection element including a second coupling member (80) configured to engage the first coupling member (40) of the cap to secure the cap to the connection element;
wherein at least a portion of one or more of the cap (20) and the connection element (60) is elastically deformable, movement of the cap in a first direction relative to the connection element causes one or more of the cap and the connection element to elastically deform to secure the cap to the connection element and movement of the cap in a second direction relative to the connection element causes one or more of the cap and the connection element to elastically deform to remove the cap from the connection element.

2. The cap assembly (10) of claim 1, wherein the first coupling member (40) of the cap (20) extends radially from the body (22) of the cap.

3. The cap assembly (10) of any of claims 1-2, wherein the second coupling member (80) of the connection element (60) extends radially from the body (62) of the connection element.

4. The cap assembly (10) of any of claims 1-3, wherein the first coupling member (40) of the cap (20) includes a recess (42a) defined by the body (22) of the cap and the second coupling member (80) of the connection element (60) includes a shoulder (82a) extending from the body (62) of the connection element (60).

5. The cap assembly (10) of any of claims 1-3, wherein the first coupling member (40) of the cap (20) includes a shoulder (42b) extending from the body (22) of the cap and the second coupling member (80) of the connection element (60) includes a recess (82b) defined by the body (62) of the connection element.

6. The cap assembly (10) of any of claims 1-5, wherein the cap (20) is formed from a first material having a first rigidity and the connection element (60) is formed from a second material having a second rigidity, and
wherein the second rigidity of the second material is greater than the first rigidity of the first material.

7. The cap assembly (10) of any of claims 1-6, wherein the body (62) of the connection element (60) defines a first opening (70) at the proximal end (64) of the body of the connection element and a second opening (72) at the distal end (66) of the body of the connection element.

8. The cap assembly (10) of claim 7, wherein the first opening (70) of the body (62) of the connection element (60) is configured to receive at least a portion of the medical device (100).

9. The cap assembly (10) of any of claims 7-8, wherein the second opening (72) of the body (62) of the connection element (60) is configured to dispense a material from the medical device (100).

10. The cap assembly (10) of any of claims 7-9, wherein the cap (20) includes an axially extending protuberance (32) configured to hermetically seal the second opening (72) of the body (62) of the connection element (60).

11. The cap assembly (10) of any of claims 1-10, wherein the body (62) of the connection element (60) includes a first section (74) having a first diameter and a second section (76) having a second diameter, and
wherein the second diameter of the second section is greater than the first diameter of the first section.

12. The cap assembly (10) of claim 11, wherein the proximal end (24) of the body (22) of the cap (20) is configured to extend to a position about the first section (74) of the body (62) of the connection element (60) when the connection element is fully-received within the cavity (28) of the cap.

13. The cap assembly (10) of any of claims 1-12, wherein the first coupling member (40) of the cap (20) is arranged at a position on the body (22) of the cap at a distance within a range of 5 mm to 10 mm from the proximal end (24) of the cap.

14. A medical device (100) including the cap assembly (10) according to any of claims 1-13.

15. A method of securing a cap (20) to a medical device (100) comprising the connection element (60), the method comprising:
providing the cap assembly (10) according to any of claims 1-13;
inserting the connection element (60) into the cavity (28) of the cap (20).
